# EUROPÄISCHE PATENTANMELDUNG

(11) **EP 1 738 783 A1**
(43) Veröffentlichungstag der Anmeldung: **03.01.2007**
(21) Anmeldenummer: 05405423.4
(22) Anmeldetag: 01.07.2005
(51) Int. Cl.: A61M 1/10, F04D 3/02, F04D 13/06

(54) **Axialpumpe mit spiralförmiger Schaufel**

(71) Anmelder: Universitätsspital Basel, 4031 Basel (CH); Sulzer Markets and Technology AG, 8401 Winterthur (CH)
(72) Erfinder: Baykut, Doan, Dr., 4056 Basel (CH); Hirt, Felix, Dr., 8400 Winterthur (CH); Baykut, Goekhan, Dr., 28213 Bremen (DE)
(74) Vertreter: Sulzer Management AG

(57) **Zusammenfassung**

Es wird eine Axialpumpe (10) vorgestellt, die ein Gehäuse (1) mit einem Einlass (2) und einem Auslass (3) und im Gehäuse einen magnetisch wirksamen Rotor (5) umfasst, der im Innern mit einem Durchlass (6) versehen ist und im Durchlass mit mindestens einer spiralförmigen Schaufel (7), wobei die Steigung der Schaufel (7) in axialer Richtung ändert. Weiter umfasst die Axialpumpe (10) einen elektromagnetischen Antrieb (4, 8) mit einem am Gehäuse angeordneten Stator (8), der mit dem magnetisch wirksamen Rotor zusammenwirkt.

## Beschreibung

Die Erfindung betrifft eine Axialpumpe gemäss Oberbegriff von Anspruch 1.

Pumpen für Anwendungen mit kleinen Druckdifferenzen von typisch 100 hPa bis 150 hPa und vergleichsweise grossen Volumenströmen, wie beispielsweise Blutpumpen, werden mit Vorteil als Axialpumpen ausgeführt. Das Dokument WO 96/18358 beschreibt eine derartige Blutpumpe mit einem rohrförmigen Pumpengehäuse, in dem ein Rotor jeweils an den Enden in einem Lager drehbar gelagert ist. Der beschriebene Rotor umfasst einen zylinderförmigen Rotorkörper und daran anschliessend einlassseitig eine Nabe und auslassseitig ein Endstück, die jeweils in einen Lagerzapfen auslaufen. Weiter sind auf dem Rotorkörper mehrere gebogene Schaufeln vorgesehen sowie im Innern des Rotorkörpers ein Permanentmagnet, um den Rotor magnetisch anzutreiben. Die vergleichsweise grosse Kontaktfläche von Nabe. Rotorkörper und Endstück mit dem Blut sowie die im Voiumenstrom angeordneten Lager fördern die Entstehung von Ablagerungen und Thromben.

Die Patentschrift US 6 527 521 B2 offenbart eine Weiterentwicklung einer Blutpumpe mit einem rohrförmigen Pumpengehäuse, in dem ein hohlzylinderförmiger Rotor angeordnet ist, der am Umfang mit Permanentmagneten versehen ist. Der Rotor ist an beiden Enden abgeschrägt und das Pumpengehäuse an den entsprechenden Stellen verjüngt, so dass der Spalt zwischen Rotor und Pumpengehäuse klein gehalten ist, Auf der äusseren Oberfläche des Pumpengehäuses sind ein Stator angeordnet, um den Rotor magnetisch anzutreiben, sowie mehrere Permanentmagnete, um den Rotor magnetisch zu lagern. An der inneren Oberfläche des Rotors ist eine spiralförmige Schaufel mit konstanter Steigung ausgebildet, derart, dass das Zentrum des Rotors offen gehalten wird.

Die axialen Blutpumpen aus dem Stand der Technik haben den Nachteil, dass entweder die Kontaktfläche mit dem Hauptvolumenstrom des Blutes wegen des geschlossenen Rotorkörpers und der Nabe vergleichsweise gross ist oder im Fall eines hohlzylinderförmigen Rotors der erzielbare Druck sehr klein ist wegen der konstanten Steigung der Schaufel und dem offenen Rotorzentrum. Zudem ist bei der letzteren Ausführung der Austrittswinkel ungünstig. Ein weiterer Nachteil der axialen Blutpumpen aus dem Stand der Technik betrifft die Lagerung des Rotors. In der in WO 96/18358 beschriebenen Blutpumpe befinden sich die Zapfenlager im Hauptvolumenstrom, was sich als ungünstig erwiesen hat. In der in US 6 527 521 B2 offenbarten Blutpumpe wird der Rotor zwar berührungslos, d.h. magnetisch gelagert, jedoch ausschliesslich passiv, was als nicht optimal eingestuft wird, insbesondere wenn Trägheitskräfte auf den Rotor wirken und/oder der Druck variiert.

Aufgabe der Erfindung ist es, eine berührungslos angetriebene Pumpe für kleine Druckdifferenzen von typisch 100 hPa, 150 hPa oder 200 hPa zur Verfügung zu stellen, die insbesondere als Blutpumpe, beispielsweise als Ventricle Assist Device geeignet ist, und die eine vergleichsweise geringe Kontaktfläche mit dem Hauptvolumenstrom aufweist.

Diese Aufgabe wird erfindungsgemäss durch die in Anspruch 1 definierte Axialpumpe gelöst.

Die erfindungsgemässe Axialpumpe umfasst ein Gehäuse mit einem Einlass und einem Auslass und im Gehäuse einen Rotor, beispielsweise einen länglich geformten Rotor, der im Innern mit einem Durchlass und im Durchlass mit mindestens einem spiralförmigen Förderelement, beispielsweise einer spiralförmigen Schaufel versehen ist, wobei sich die Steigung des Förderelements in axialer Richtung ändert. Weiter umfasst die Axialpumpe einen elektromagnetischen Antrieb, um den Rotor anzutreiben, wobei am Gehäuse ein Stator vorgesehen und der Rotor oder ein Teil desselben magnetisch wirksam ausgebildet ist.

In einer bevorzugten Ausführungsvariante nimmt die Steigung des Förderelements in Richtung zum Auslass hin zu, und in einer weiteren bevorzugten Ausführungsvariante ist das Zentrum des Rotors über mindestens einen Teil der Rotorlänge offen. Vorzugsweise ist das Zentrum des Rotors zum Einlass hin offen und/oder zum Auslass hin geschlossen.

In einer weiteren bevorzugten Ausführungsvariante umfasst die Axialpumpe eine zusätzliche Pumpenstufe, deren Zentrum zum Auslass hin geschlossen ist, insbesondere eine zweite Pumpenstufe, die axial oder halbaxial ausgeführt und/oder im Zentrum mit einer Nabe versehen ist.

Vorzugsweise weist das Förderelement und/oder eine Kante desselben ein abgerundetes oder zugespitztes Profil auf. Vorzugsweise umfasst das Förderelement eine oder mehrere Schaufeln und/oder Löcher und/oder Schlitze und/oder lokale Erhöhungen und/oder lokale Vertiefungen.

In einer weiteren bevorzugten Ausführungsvariante ist im Betrieb zwischen dem Gehäuse und dem Rotor ein Spalt von mindestens 0.3 mm Breite, insbesondere mindestens 0.6 mm Breite vorgesehen. Vorzugsweise sind an der äusseren Oberfläche des Rotors mindestens ein Aussenförderelement vorgesehen, das beispielsweise eine oder mehrere Schaufeln und/oder Löcher und/oder Schlitze und/oder lokale Erhöhungen und/oder lokale Vertiefungen umfasst.

In einer weiteren bevorzugten Ausführungsvariante enthält mindestens ein Teil des Rotors ein permanent magnetisches oder weichmagnetisches Material oder der Rotor umfasst mindestens eine Kurzschlusswindung. Vorzugsweise ist der elektromagnetische Antrieb gleichzeitig als Magnetlager ausgebildet. Vorzugsweise sind in der Axialpumpe mindestens zwei axial versetzt angeordnete Magnetlager vorgesehen, wobei beispielsweise mindestens eines der Magnetlager im einlassseitigen und/oder auslassseitigen Endbereich des Rotors angeordnet ist.

In einer weiteren bevorzugten Ausführungsvariante umfasst die Axialpumpe mindestens ein Leitelement, das im Einlassbereich oder zwischen dem Gehäuse und dem Rotor, oder im Auslassbereich am Gehäuse angeordnet ist. Vorzugsweise umfasst die Axialpumpe jeweils mindestens zwei im Einlassbereich und/oder im Auslassbereich am Gehäuse angeordnete Leitelemente.

In einer weiteren bevorzugten Ausführungsvariante umfasst die Axialpumpe eine oder zwei ringförmige Rotorkappen, die beispielsweise an Halteelementen, vorzugsweise Halteschaufeln befestigt sind, und die in Flussrichtung vor und/oder nach dem Rotor angeordnet sind.

In einer weiteren bevorzugten Ausführungsvariante ist am Gehäuse mindestens ein Kupplungsteil zur Verbindung der Axialpumpe mit einer Einlasskanüle und/oder Auslasskanüte vorgesehen.

Die erfindungsgemässe Axialpumpe hat den Vorteil, dass Druckdifferenzen von beispielsweise 100 hPa, 150 hPa oder 200 hPa erzielt werden können, und dass sie als implantierbare Blutpumpe eingesetzt werden kann. Vorteilhaft ist auch, dass die erfindungsgemässe Axialpumpe eine vergleichsweise geringe Kontaktfläche mit dem Hauptvolumenstrom aufweist, und dass die Blutkörperchen nicht auf den Widerstand einer Nabe stossen. Weiter ist durch den vergleichsweise breiten Spalt zwischen Gehäuse und Rotor eine bessere Umspülung möglich.

Weitere vorteilhafte Ausführungsformen gehen aus den abhängigen Ansprüchen und der Zeichnung hervor.

Im Folgenden wird die Erfindung an Hand der Ausführungsbeispiele und an Hand der Zeichnung näher erläutert. Es zeigen:
- Fig. 1: Längsschnitt eines Ausführungsbeispiels einer Axialpumpe gemäss der vorliegenden Erfindung,
- Fig. 2A, B: Querschnitte durch den Einlassbereich und Auslassbereich der Axialpumpe gemäss dem in Fig. 1 gezeigten Ausführungsbeispiel,
- Fig. 3: eine Ausführungsvariante mit mehreren Förderelementen im Innern des Rotors,
- Fig. 4: eine weitere Ausführungsvariante mit einem vergleichsweise breiten Spalt zwischen Gehäuse und Rotor,
- Fig. 5A, B: Querschnitte durch den Einlassbereich und Auslassbereich der Axialpumpe gemäss der in Fig. 4 gezeigten Ausführungsvariante,
- Fig. 6: Längsschnitt eines zweiten Ausführungsbeispiels einer Axialpumpe gemäss der vorliegenden Erfindung,
- Fig. 7A, B: Querschnitte durch den Einlassbereich und Auslassbereich der Axialpumpe gemäss dem in Fig. 6 gezeigten Ausführungsbeispiel,
- Fig. 8: eine Ausführungsvariante zu dem in Fig. 6 gezeigten Ausführungsbeispiel mit nur einem Förderelement im Innern des Rotors,
- Fig. 9: Längsschnitt durch das Gehäuse des in Fig. 6 gezeigten Ausführungsbeispiels,
- Fig. 10: Aussenansicht einer Ausführungsvariante mit Kupplungsteilen,
- Fig. 11: Längsschnitt eines weiteren Ausführungsbeispiels einer Axialpumpe gemäss der vorliegenden Erfindung, und
- Fig. 12: Querschnitte durch den Auslassbereich der Axialpumpe gemäss dem in Fig. 11 gezeigten Ausführungsbeispiel.

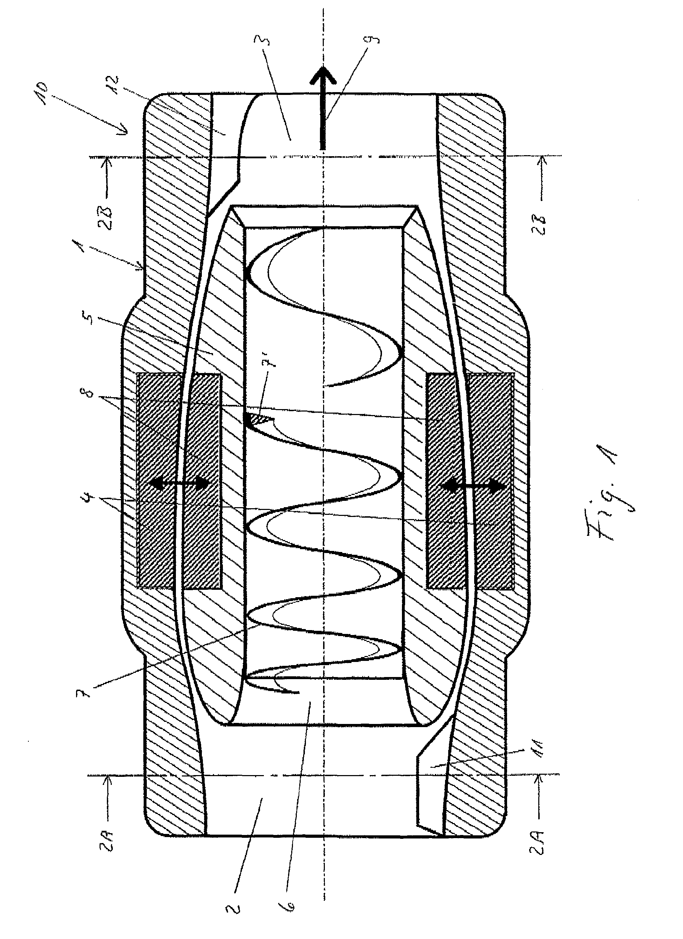

Fig. 1 zeigt einen Längsschnitt durch ein Ausführungsbeispiel einer Axialpumpe gemäss der vorliegenden Erfindung. Im gezeigten Ausführungsbeispiel umfasst die Axialpumpe 10 ein Gehäuse 1 mit einem Einlass 2 und einem Auslass 3 und im Gehäuse einen drehbar angeordneten Rotor 5, beispielsweise einen länglich geformten Rotor. Das Gehäuse 1 ist mit Vorteil, wie in Fig. 1 gezeigt, als länglicher Hohlkörper ausgebildet mit einer kreisförmigen Öffnung im Innern, die beispielsweise im Bereich des Rotors einen veränderten, z.B. einen vergrösserten, Durchmesser aufweisen kann. Pfeil 9 bezeichnet die Durchlassrichtung der Axialpumpe.

Der Rotor 5 ist im Innern mit einem Durchlass 6 versehen und kann z.B. als Hohlzylinder ausgebildet sein, oder, wie in Fig. 1 gezeigt, eine strömungsmässig angepasste Form aufweisen. Im Durchlass 6 ist auf der inneren Oberfläche des Rotors ein spiralförmiges Förderelement 7 angeordnet, das beispielsweise als spiralförmige Schaufel ausgebildet ist, wobei sich die Steigung des Förderelements in axialer Richtung ändert. Bei Bedarf können im Durchlass 6 auch mehrere Förderelemente vorgesehen werden.

Weiter enthält die Axialpumpe des in Fig. 1 gezeigten Ausführungsbeispiels einen elektromagnetischen Antrieb, vorzugsweise um den Rotor 5 magnetisch, d.h. berührungslos anzutreiben, wobei am Gehäuse 1 ein Stator 4 vorgesehen und der Rotor oder ein Teil 8 desselben magnetisch wirksam ausgebildet ist, beispielsweise indem der Rotor oder ein Teil desselben ein permanent magnetisches oder weichmagnetisches Material oder mindestens eine Kurzschlusswindung enthält. Der Stator 4 umfasst beispielsweise mehrere Wicklungen, die auf einem weichmagnetischen Kern angeordnet sind, um im Betrieb ein Drehfeld zu erzeugen. In einer vorteilhaften Ausführungsform ist der elektromagnetische Antrieb gleichzeitig als Magnetlager ausgebildet.

Die Axialpumpe gemäss der vorliegenden Erfindung eignet sich beispielsweise als Blutpumpe, speziell als implantierbare Blutpumpe. Vorteilhafterweise werden die mit Blut in Kontakt kommenden Oberflächen teilweise oder ganz mit einer blutverträglichen Beschichtung versehen, z.B. mit einer Beschichtung, die Diamondlike Carbon (DLC) enthält.

In einer vorteilhaften Ausführungsvariante nimmt die Steigung, oder wahlweise der Steigungswinkel oder die Ganghöhe des Förderelements 7 in Richtung zum Auslass 3 hin zu, womit beispielsweise der Ausgangsdruck erhöht und/oder der Austrittswinkel vergrössert werden kann. In einer weiteren vorteilhaften Ausführungsvariante ist das Zentrum des Rotors über mindestens einen Teil der Rotorlänge offen, wobei der offengelassene Querschnitt über die Länge des Rotors variieren kann, beispielsweise indem der offengelassene Querschnitt zum Auslass hin abnimmt, so dass der offengelassene Querschnitt am auslassseitigen Ende des Rotors vergleichsweise klein ist oder sich das Förderelement 7 am auslassseitigen Ende ins Zentrum des Rotors erstreckt. In einer vorteilhaften Ausführungsform ist das Zentrum des Rotors zum Einlass hin offen und/oder zum Auslass hin geschlossen.

Das Förderelement 7 und/oder eine Kante desselben weist mit Vorteil ein abgerundetes oder zugespitztes Profil 7' auf, beispielsweise ein Dreiecksprofil, das zur Rotorachse hin zugespitzt ist oder ein zur Rotorachse hin abgerundetes Profil oder wahlweise abgerundete und/oder abgeschrägte Ein- und Austrittskanten, In einer weiteren vorteilhaften Ausführungsform umfasst das Förderelement wahlweise Löcher, Schlitze, lokale Erhöhungen und/oder lokale Vertiefungen.

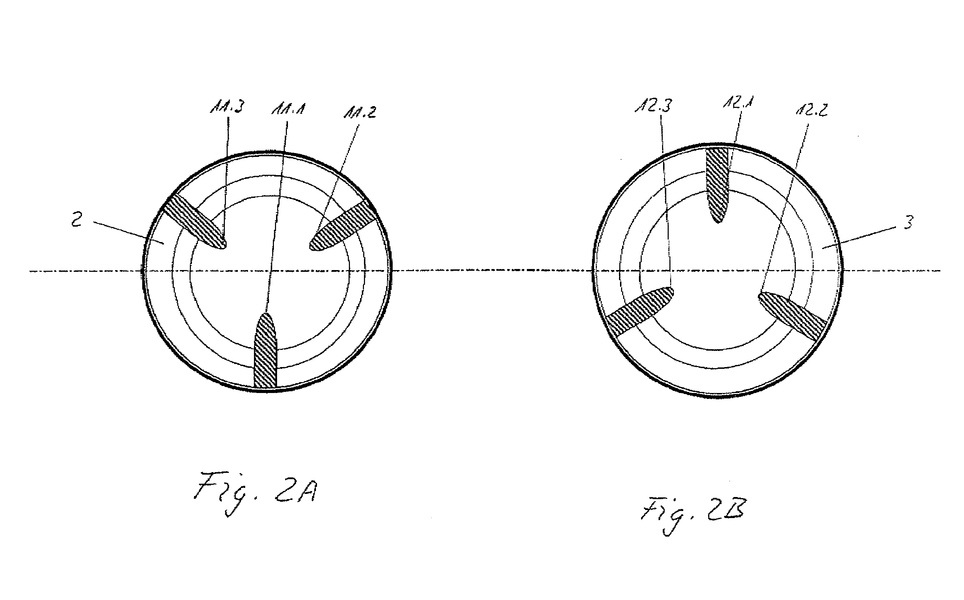

Die Figuren 2A und 2B zeigen Querschnitte durch den Einlassbereich und Auslassbereich der Axialpumpe gemäss dem in Fig. 1 gezeigten Ausführungsbeispiel. In diesem Ausführungsbeispiel sind im Einlassbereich und Auslassbereich der Axialpumpe jeweils drei axiale Leitschaufeln 11.1-11,3, 12.1-12.3 angeordnet. Selbstverständlich ist es auch möglich, entweder nur im Einlass- oder nur im Auslassbereich Leitelemente anzuordnen. Ebenso ist die Zahl der Leitelemente wählbar. Typisch werden Anordnungen von zwei bis vier Leitschaufeln vorgesehen. Im Weiteren können auch die Form und das Profil der Leitelemente den besonderen Verhältnissen angepasst werden, beispielsweise indem axiale, schräg laufende oder gebogene Leitschaufeln vorgesehen werden oder Leitschaufeln oder Leitschaufelkanten mit abgerundetem, auslaufendem oder zugespitztem Profil.

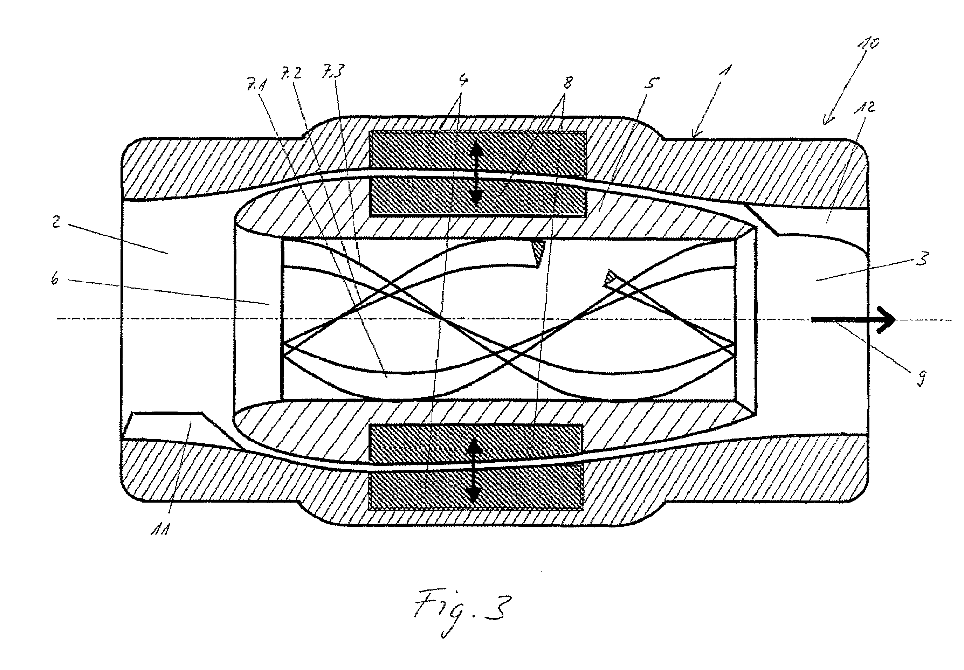

In der in Fig. 3 gezeigten Ausführungsvariante ist der Rotor 5 im Innern mit mehreren Förderelementen 7.1-7.3 versehen. In diesem Fall kann es vorteilhaft sein, die Steigung der Förderelemente gegenüber dem Ausführungsbeispiel mit nur einem Förderelement zu erhöhen.

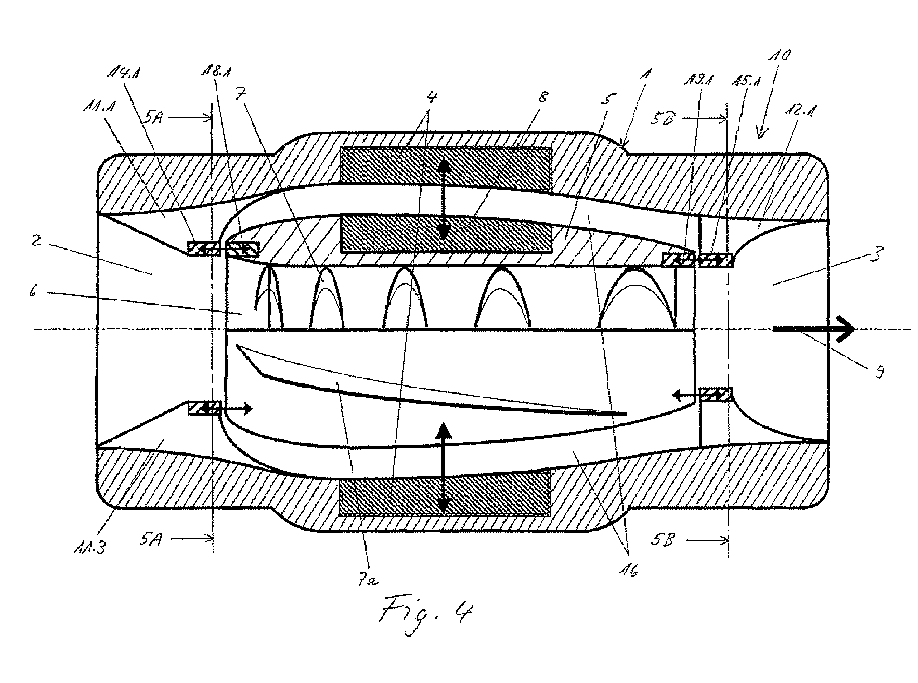

Fig. 4 zeigt eine weitere Ausführungsvariante mit einem im Betrieb vergleichsweise breiten Spalt 16 zwischen Gehäuse 1 und Rotor 5. beispielsweise mit einem Spalt von mindestens 0.3 mm Breite, insbesondere mindestens 0.6 mm oder 1 mm Breite. In einer vorteilhaften Ausführungsform sind an der äusseren Oberfläche des Rotors 5 mindestens ein Aussenförderelement 7a vorgesehen, beispielsweise in Form von Schaufeln und/oder Löchern und/oder Schlitzen und/oder lokalen Erhöhungen und/oder lokalen Vertiefungen.

In einer vorteilhaften Ausführungsvariante können in der Axialpumpe eines oder mehrere Magnetlager vorgesehen werden, um den Rotor 5 berührungsfrei zu lagern, beispielsweise indem der elektromagnetische Antrieb 4, 8 gleichzeitig als Magnetlager ausgebildet wird und/oder indem die Axialpumpe mit einem oder mehreren axial versetzt angeordneten Magnetlagern versehen wird. In der in Fig. 4 gezeigten Ausführungsvariante sind jeweils im einlassseitigen und/oder auslassseitigen Endbereich des Rotors 5 Magnetlager {14.1, 18.1}, {15.1, 19.1} angeordnet, die beispielsweise als passive Magnetlager ausgebildet sein können. Die Leitschaufeln 11.1-11.4 und 12.1-12.4 sind dazu in dem an den Rotor grenzenden Teil mit Permanentmagneten 14.1 und 15.1 versehen, während der Rotor im einlassseitigen und auslassseitigen Endbereich mit jeweils einem oder mehreren Permanentmagneten 18.1, 19.1 bestückt ist. An Stelle von mehreren Permanentmagneten in jedem Rotorendbereich kann jeweils auch ein ringförmiger Permanentmagnet verwendet werden. Bedarfsweise können die Leitschaufeln 11.1-11.4 und 12.1-12.4 auch mit Elektromagneten 14.1 und 15.1 versehen werden, mit denen die Lage des Rotors 5 aktiv stabilisiert werden kann.

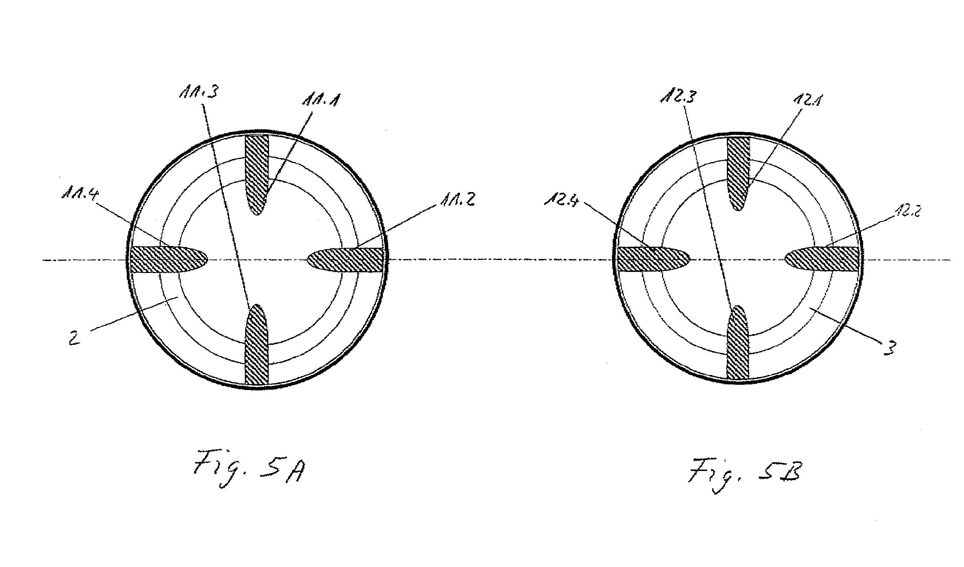

Die Figuren 5A und 5B zeigen Querschnitte durch den Einlassbereich und Auslassbereich der Axialpumpe gemäss der in Fig. 4 gezeigten Ausführungsvariante. In dieser Ausführungsvariante sind im Einlassbereich und Auslassbereich der Axialpumpe jeweils vier Leitschaufeln 11.1-11.4, 12.1-12.4 angeordnet.

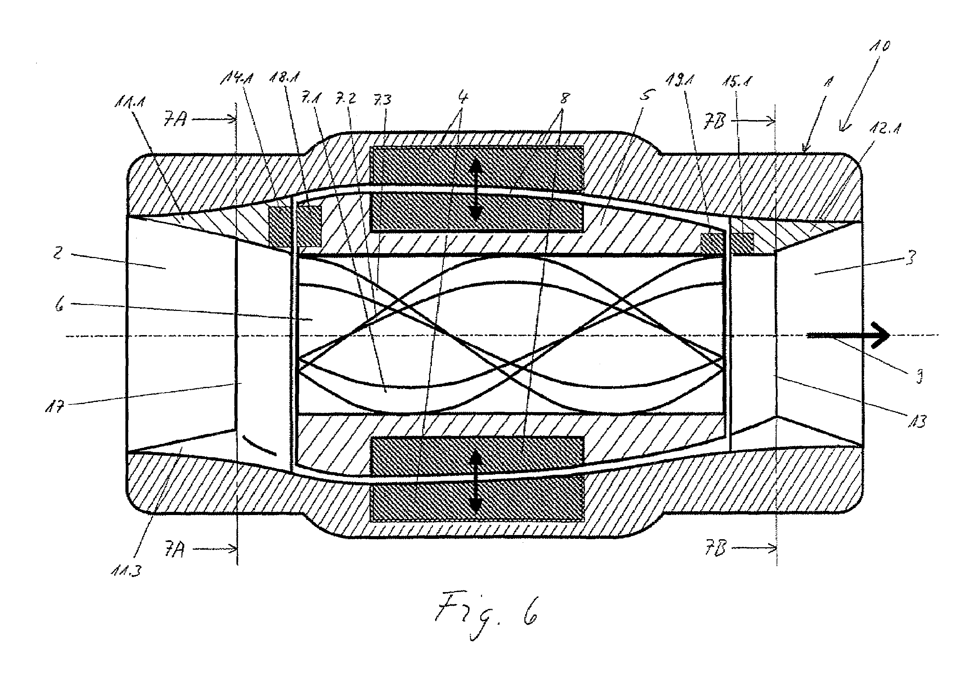

Fig. 6 zeigt einen Längsschnitt durch ein zweites Ausführungsbeispiel einer Axialpumpe gemäss der vorliegenden Erfindung. Das zweite Ausführungsbeispiel unterscheidet sich vom ersten Ausführungsbeispiel gemäss Fig. 1 insbesondere dadurch, dass in Flussrichtung vor und/oder nach dem Rotor 5 Rotorkappen 17, 13 angeordnet sind, die vorteilhafterweise ringförmig ausgebildet sind. Die übrigen Merkmale des zweiten Ausführungsbeispiels sind bereits aus der Beschreibung des ersten Ausführungsbeispiels bekannt mit Ausnahme der Anzahl der Förderelemente im Innern des Rotors, die im Zusammenhang mit der in Fig. 3 gezeigten Ausführungsvariante beschrieben wurde, und mit Ausnahme der Magnetlager am einlassseitigen und auslassseitigen Ende des Rotors, die im Zusammenhang mit der in Fig. 4 gezeigten Ausführungsvariante erläutert wurden.

Die Rotorkappen 13, 17 haben den Vorteil, dass darin grössere Permanentmagnete 14.1 und 15.1 angeordnet werden können, beispielsweise ein ringförmiger Permanentmagnet 14,1 in der einlassseitigen Rotorkappe 17, der grössere Axialkräfte aufnehmen kann. Da bei berührungsloser Lagerung des Rotors die sich aufgrund der Druckdifferenz zwischen Einlass und Auslass ergebende Axialkraft vollständig von den Magnetlagern aufgenommen werden muss, lässt sich mit der Axialpumpe gemäss dem zweiten Ausführungsbeispiel eine grössere Druckdifferenz erzeugen. Bei Bedarf können an Stelle der Permanentmagnete auch Elektromagnete in den Rotorkappen vorgesehen werden, mit denen die Lage des Rotors aktiv stabilisiert werden kann.

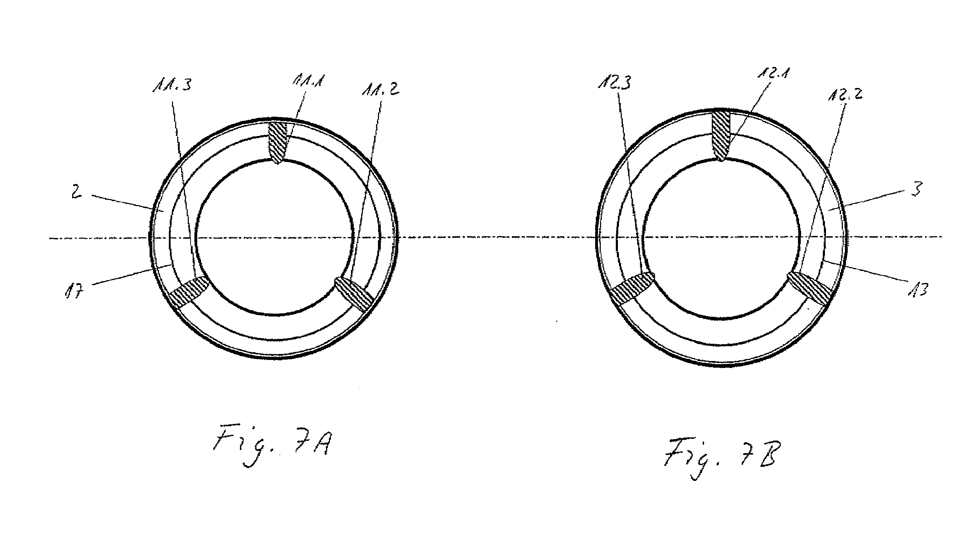

Die Figuren 7A und 7B zeigen Querschnitte durch den Einlassbereich und Auslassbereich der Axialpumpe gemäss dem in Fig. 6 gezeigten Ausführungsbeispiel. In diesem Ausführungsbeispiel sind im Einlassbereich und Auslassbereich der Axialpumpe jeweils drei Leitschaufeln 11.1-11.3, 12.1-12.3 angeordnet, die z.B. gleichzeitig als Halteschaufeln für die Rotorkappen 13, 17 ausgebildet sein können.

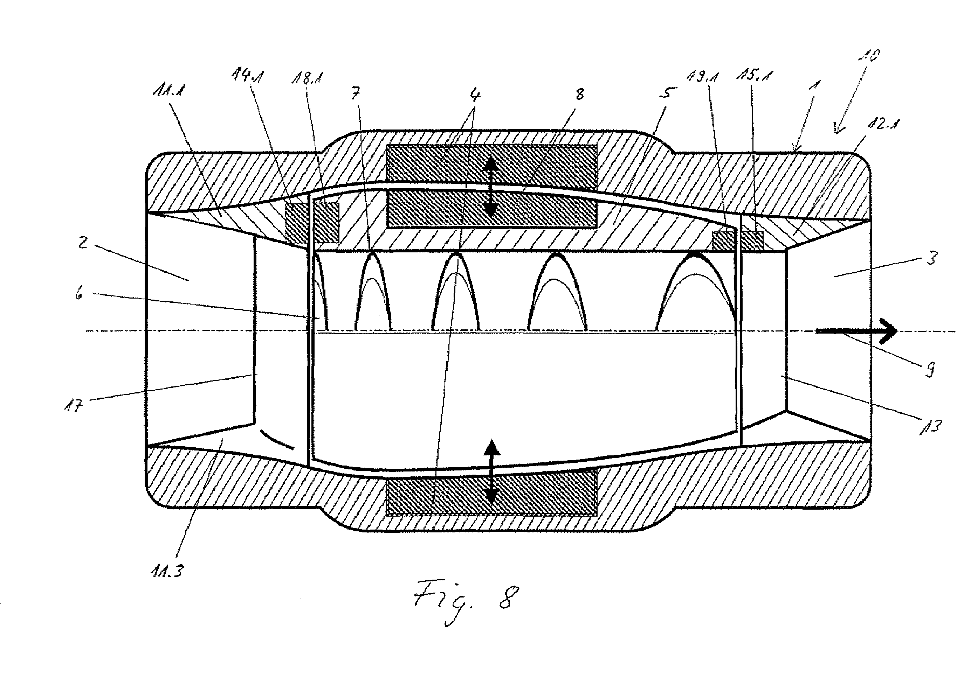

In der in Fig. 8 gezeigten Ausführungsvariante ist der Rotor 5 im Innern mit einem einzigen Förderelement 7 versehen. Die übrigen Merkmale entsprechen denjenigen aus dem zweiten Ausführungsbeispiel gemäss Fig. 6.

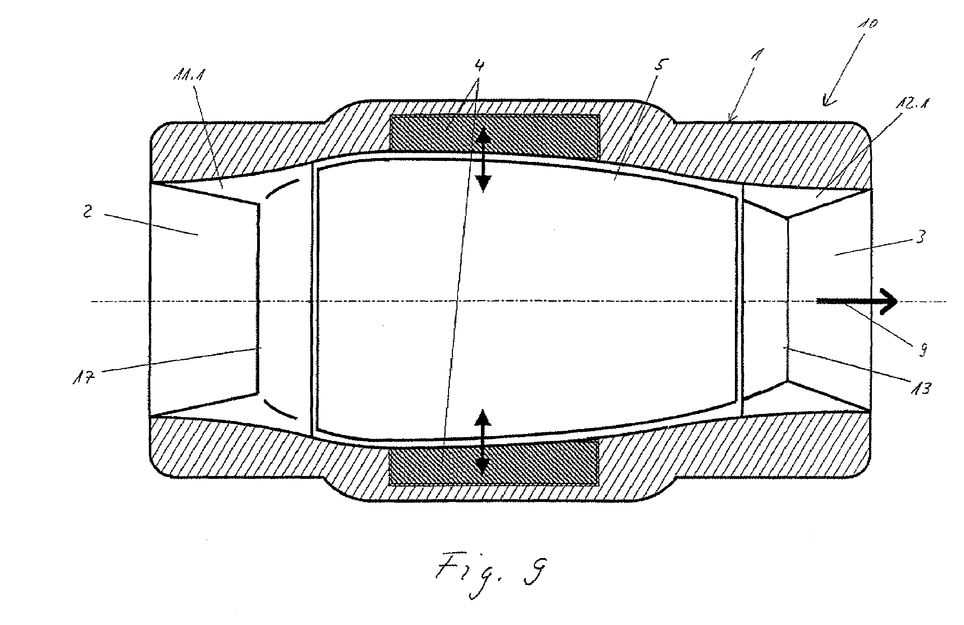

Fig. 9 zeigt eine weitere Ansicht des zweiten Ausführungsbeispiels gemäss Fig. 6. In dieser Ansicht ist das Gehäuse im Längsschnitt dargestellt, während der Rotor 5, die Rotorkappen 13, 17 und die Leitschaufeln 11.1, 12.1 in der Aufsicht wiedergegeben sind.

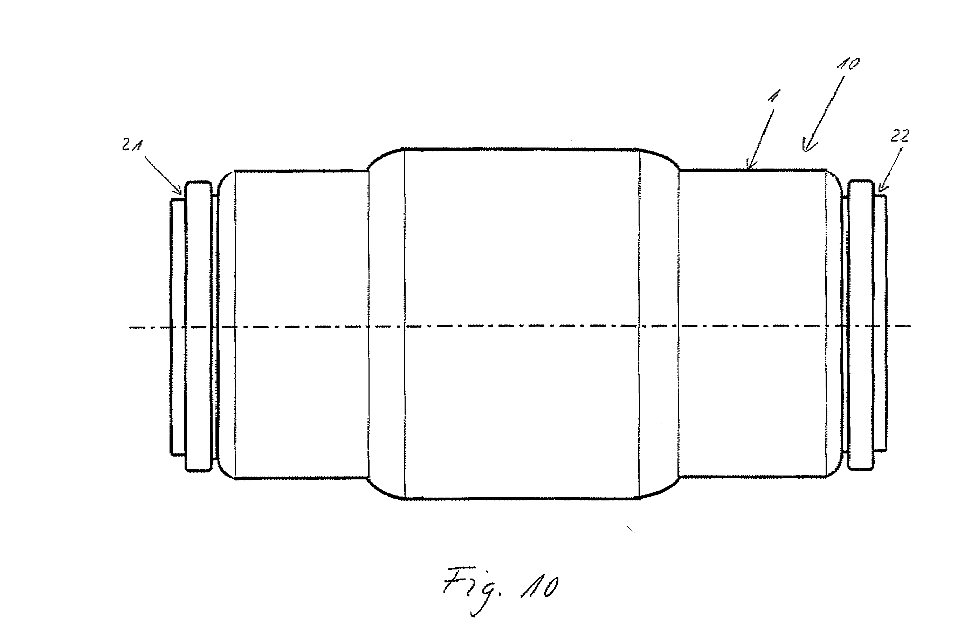

Fig. 10 zeigt eine weitere bevorzugte Ausführungsvariante in dieser Ausführungsvariante umfasst die Axialpumpe 10 ein Gehäuse 1 und einlassseitig und auslassseitig jeweils einen Kupplungsteil 21, 22, an den eine Einlass- beziehungsweise Auslasskanüle angeschlossen werden kann.

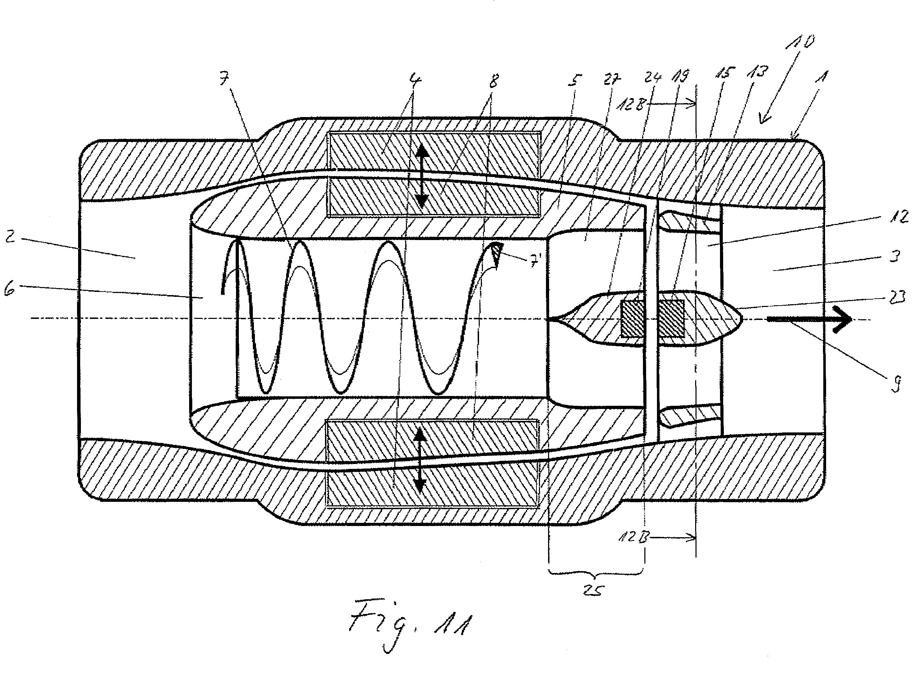

Fig. 11 zeigt einen Längsschnitt durch ein weiteres Ausführungsbeispiel einer Axialpumpe gemäss der vorliegenden Erfindung. Das gezeigte Ausführungsbeispiel unterscheidet sich vom ersten Ausführungsbeispiel gemäss Fig. 1 insbesondere dadurch, dass die Pumpe 10 eine zusätzliche Pumpenstufe 25 umfasst, deren Zentrum zum Auslass 3 hin geschlossen ist. Die zusätzliche Pumpenstufe 25 kann beispielsweise, wie in Fig. 11 gezeigt, im Rotor 5 ausgebildet sein. Es ist aber auch möglich für die zusätzliche Pumpenstufe einen eigenen Rotor vorzusehen, der bei Bedarf mit einem eigenen Antrieb ausgestattet sein kann. Wie in Fig. 11 gezeigt, kann die zusätzliche Pumpenstufe eine Nabe 24 und ein oder mehrere Förderelemente 27 umfassen, die z.B. als Schaufeln ausgebildet sein können. Die zusätzliche Pumpenstufe kann axial oder halbaxial ausgeführt werden. Eine derartige Pumpe mit zusätzlicher Pumpenstufe stellt eine eigenständige und unabhängige Erfindung dar, die in einer eigenen Patentanmeldung geschützt werden kann.

In einer bevorzugten Ausführungsvariante umfasst die Pumpe mit zusätzlicher Pumpenstufe auslassseitig mindestens ein Leitelement, beispielsweise einen Leitring 13 mit Halteelementen 12, die als Leitschaufeln ausgebildet sein können. Bedarfsweise kann im Zentrum eine Kappe 23 an den Halteelementen beziehungsweise Leitschaufeln vorgesehen werden.

in einer weiteren bevorzugten Ausführungsvariante ist in der Nabe 24 und in der Kappe 23 ein Magnetlager 19, 15 und/oder eine elektromagnetische Antriebsvorrichtung vorgesehen.

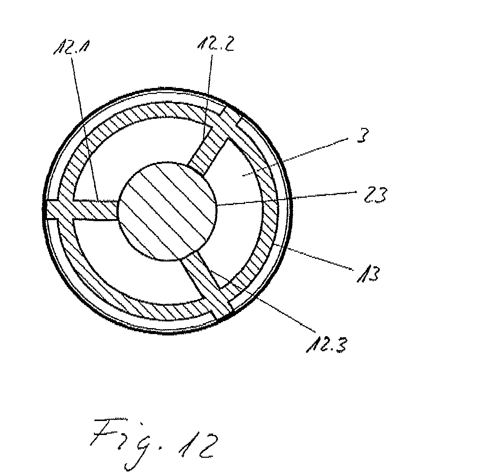

Fig. 12 zeigt einen Querschnitt durch den Auslassbereich der Axialpumpe gemäss dem in Fig. 11 gezeigten Ausführungsbeispiel. In diesem Ausführungsbeispiel sind im Auslassbereich der Axialpumpe jeweils drei Leitschaufeln 12.1-12.3 angeordnet, die auch als Halteelemente für den Leitring 13 und die Kappe 23 dienen. Die Anzahl der Halteelemente beziehungsweise Leitschaufeln kann in einem weiten Bereich frei gewählt werden. Typisch werden Anordnungen von zwei bis vier Leitschaufeln vorgesehen. Im Weiteren können auch die Form und das Profil der Halteelemente beziehungsweise Leitschaufeln den besonderen Verhältnissen angepasst werden, beispielsweise indem axiale, schräg laufende oder gebogene Leitschaufeln vorgesehen werden oder Leitschaufeln oder Leitschaufelkanten mit abgerundetem, auslaufendem oder zugespitztem Profil.

Mit der erfindungsgemässen Axialpumpe können je nach Ausführung Druckdifferenzen von 150 hPa und mehr erzeugt werden. Dank des berührungslosen Antriebs und der berührungslosen Lagerung des Rotors ist die Axialpumpe als Blutpumpe einsstzbar. Vorteilhaft ist in diesem Zusammenhang, dass die erfindungsgemässe Axialpumpe eine vergleichsweise geringe Kontaktfläche mit dem Hauptvolumenstrom aufweist, und dass durch die beschriebene Ausgestaltung des Spaltes zwischen Gehäuse und Rotor eine bessere Umspülung des Rotors möglich ist.

## Patentansprüche

1. Axialpumpe umfassend ein Gehäuse (1) mit einem Einlass (2) und einem Auslass (3), im Gehäuse einen Rotor (5), insbesondere einen länglich geformten Rotor, der im Innern mit einem Durchlass (6) versehen ist und im Durchlass (6) mit mindestens einem spiralförmigen Förderelement (7), und einen elektromagnetischen Antrieb (4, 8), um den Rotor (5) anzutreiben, derart, dass am Gehäuse (1) ein Stator (4) vorgesehen und der Rotor oder ein Teil (8) desselben magnetisch wirksam ausgebildet ist, **dadurch gekennzeichnet, dass** sich die Steigung des Förderelements (7) in axialer Richtung ändert.

2. Axialpumpe nach Abspruch 1, wobei die Steigung des Förderelements (7) in Richtung zum Auslass (3) hin zunimmt und/oder wobei das Zentrum des Rotors (5) über mindestens einen Teil der Rotorlänge offen ist.

3. Axialpumpe nach einem der Ansprüche 1 oder 2, wobei das Zentrum des Rotors (5) zum Einlass hin offen und zum Auslass hin geschlossen ist.

4. Axialpumpe nach einem der Ansprüche 1 bis 3, umfassend eine zusätzliche Pumpenstufe (25), deren Zentrum zum Auslass (3) hin geschlossen ist, insbesondere eine zweite Pumpenstufe, die axial oder halbaxial ausgeführt und/oder im Zentrum mit einer Nabe (24) versehen ist.

5. Axialpumpe nach einem der vorangehenden Ansprüche, wobei das Förderelement (7) und/oder eine Kante desselben ein abgerundetes oder zugespitztes Profil (7') aufweist und/oder wobei das Förderelement (7) eine oder mehrere Schaufeln und/oder Löcher und/oder Schlitze und/oder lokale Erhöhungen und/oder lokale Vertiefungen umfasst.

6. Axialpumpe nach einem der vorangehenden Ansprüche, wobei im Betrieb zwischen dem Gehäuse (1) und dem Rotor (5) ein Spalt (16) von mindestens 0.3 mm Breite, insbesondere mindestens 0.6 mm Breite vorgesehen ist.

7. Axialpumpe nach einem der vorangehenden Ansprüche, wobei an der äusseren Oberfläche des Rotors mindestens ein Aussenförderelement (7a) vorgesehen ist, das insbesondere eine oder mehrere Schaufeln und/oder Löcher und/oder Schlitze und/oder lokale Erhöhungen und/oder lokale Vertiefungen umfasst.

8. Axialpumpe nach einem der vorangehenden Ansprüche, wobei mindestens ein Teil (8) des Rotors ein permanent magnetisches oder weichmagnetisches Material enthält, oder wobei der Rotor (5) mindestens eine Kurzschlusswindung umfasst.

9. Axialpumpe nach einem der vorangehenden Ansprüche, wobei der elektromagnetische Antrieb (4, 8) gleichzeitig als Magnetlager ausgebildet ist und/oder wobei in der Axialpumpe mindestens zwei axial versetzt angeordnete Magnetlager (14.1, 18.1 sowie 15.1. 19.1) vorgesehen sind, indem insbesondere im einlassseitigen und/oder auslassseitigun Endbereich des Rotors mindestens ein Magnetlager angeordnet ist.

10. Axialpumpe nach einem der vorangehenden Ansprüche umfassend mindestens ein Leitelement (11, 11.1-11.4, 12, 12.1-12.4) das im Einlassbereich oder zwischen dem Gehäuse und dem Rotor, oder im Auslassbereich am Gehäuse angeordnet ist, insbesondere Axialpumpe mit jeweils mindestens zwei im Einlassbereich und/oder im Auslassbereich am Gehäuse angeordneten Leitelementen.

11. Axialpumpe nach einem der vorangehenden Ansprüche umfassend eine oder zwei ringförmige Rotorkappen (13, 17), insbesondere an Halteelementen befestigte Rotorkappen, welche in Flussrichtung vor und/oder nach dem Rotor angeordnet sind.

12. Axialpumpe nach einem der vorangehenden Ansprüche, wobei am Gehäuse mindestens ein Kupplungsteil (21, 22) zur Verbindung der Axialpumpe mit einer Einlasskanüle und/oder Auslasskanüte vorgesehen ist.
